# EUROPEAN PATENT SPECIFICATION

(11) **EP 0 547 102 B1**
(45) Date of publication and mention of the grant of the patent: **08.07.1998**
(21) Application number: 91915661.2
(22) Date of filing: 29.08.1991
(51) Int. Cl.: C07K 1/00, C07K 1/08, C12P 21/02

(54) **SOLUBILIZATION OF PROTEINS IN ACTIVE FORMS**
IN LÖSUNG BRINGEN VON PROTEINEN IN AKTIVER FORM
SOLUBILISATION DE PROTEINES SOUS DES FORMES ACTIVES

(30) Priority: 05.09.1990 AU 2159/90
(43) Date of publication of application: 23.06.1993
(73) Proprietor: SOUTHERN CROSS BIOTECH PTY.LTD., Toorak, Victoria 3142 (AU)
(72) Inventor: PURI, Nirdosh, Kumar, Nebraska 68516 (US); CRIVELLI, Enzo, Hawthorn East, VIC 3123 (AU)
(74) Representative: Harding, Richard Patrick
(86) International application number: AU9100395
(87) International publication number: WO9204382

(56) References cited:
- EP-A- 0 295 859
- AU-A- 1 141 288
- AU-A- 1 712 488
- AU-A- 2 257 488
- AU-A- 2 284 188
- AU-A- 3 380 484
- AU-A- 4 132 189
- AU-A- 4 338 589
- AU-A- 4 862 090
- AU-A- 4 862 190
- AU-A- 5 232 886
- AU-A- 6 591 686
- AU-A- 6 599 386
- AU-A- 6 701 890
- AU-A- 7 119 087
- RUDOLF, R. and FUCHS, I., "Influence of Glutathione on the Reactivation of Enzymes containing Cysteine or Cystine", Hoppe-Seylers Z Physiol. Chem., V364(7) pages 813-820, 1983, page 813, see pages 816-817.
- PERRAUDIN, J.-P; TORCHIA, T.E.; WETLAUFER, D.B., "Multiple Parameter Kinetic Studies of the Oxidative Folding of Reduced Lysozyme", Journal of Biological Chemistry, V 258 (19) pages 11834-11839, 1983, see "Materials and Methods".
- LIGHT, A; DUDA, C.J.; ODORZYNSKI, J.W.; MOORE, W.G.J. "Refolding of Serine Proteinases", Journal of Cellular Biochemistry, V 31(1), pages 19-26, 1986, see "Materials and Methods".
- LIGHT, A.; HIGAKI, J.N. "Detection of Intermediate Species in the Refolding of Bovine Trypsinogen", Biochemistry, V 26(17), pages 5556-5564, 1987, page 5557, see "Experimental Procedures".
- WETLAUFER, D.B.; BRANCA, P.A.; CHEN, G.X. "The Oxidative Folding of Proteins by Disulphide plus Thiol does not correllate with Redox Potential", Protein Engineering, Vol. 1 (2), pages 141-146, 1987, see "Materials and Methods".
- GOLDENBERG, D.P., "Kinetic Analysis of the Folding and Unfolding of a Mutant form of Bovine Pancreatic Trypsin Inhibitor", Biochemistry, V 27(7), pages 2481-2489, 1988, see "Experimental Procedures".
- HIROSE, M.; AKUTA, T.; TAKAHASHI, N., "Renaturation of Ovotransferrin under 2-step Conditions allowing Primary Folding of the Fully Reduced Form and the Subsequent Regeneration of the Intramolecular Disulfides", Journal of Biological Chemistry, V 264 (28), pages 16867-16872, 1989, 5 October 90 (05.10.90), see "Experimental Procedures".
- LYLES, M.M.; GILBERT, H.F., "Catalysis of the Oxidative Folding of Ribonuclease A by Protein Disulfide Isomerase; Dependence of the Rate on the Composition of the Redox Buffer", Biochemistry, V 30 (3), pages 613-619, 1991, see "Experimental Procedures".

## Description

The present invention relates to a new method for the preparation of a protein in a biologically active form in increased yield.

Recombinant DNA technology provides potentially extremely valuable means of synthesizing large amounts of desirable eukaryotic (usually mammalian) proteins such as hormones, interferons, and enzymes. Although it has proved to be relatively easy to manipulate organisms such as bacteria in order to produce the desired protein, the host organism does not normally secrete the protein product into the culture medium. Thus lysis of the organisms (for example E.coli bacteria) followed by isolation of the desired protein, often in the form of insoluble inclusion bodies, is usually necessary.

A protein may exist as a chain of aminoacids linked by peptide bonds. In the normal biologically active form of such a protein or its native form as it is referred to, the chain is folded into a thermodynamically preferred three dimensional conformation, the structure of which is maintained by various combinations of inter- and/or intra-atomic forces such as hydrogen bonding, hydrophobic interactions, charge (ionic) interactions or covalent, that is, inter- or intra-molecular disulphide bonding.

In the prior art, problems of aggregation and/or insolubility encountered due to aberrant (non-native) disulphide bond formation during the in vitro refolding procedures used to renature partially or fully reduced and unfolded disulphide bond containing recombinant proteins, has meant that the yield of correctly refolded proteins remains generally low. Although various refolding procedures have been devised in an attempt to overcome those difficulties, there is no generally applicable method giving high yields of biologically active disulphide bonded proteins produced by recombinant means.

In copending Australian Patent Application 66874/86 applicants have described a method for the recovery of proteins in a soluble form from an insoluble protein source such as bacterial inclusion bodies, utilising cationic surfactants. Whilst this process allows for the efficient recovery of proteins in a soluble and reversibly denatured form, in particular proteins that are dependent upon inter- or intra-chain disulphide bonding may not exhibit their normal biological activity until refolded into a native state. During the refolding and concomitant formation of disulphide bonds the overall yield of correctly disulphide bonded, that is, biologically active protein is relatively low, for example of the order of 5-15%.

Accordingly, it is an object of the present invention to overcome, or at least alleviate, one or more of the difficulties related to the prior art.

Accordingly, in the first aspect of the present invention, there is provided a method of preparing a protein in a physiologically active form, which method includes
providing .
a source of protein in an at least partially solubilized form obtained by solubilizing an insoluble protein with a cationic surfactant denaturing agent in the absence of additional denaturing agents to form an at least partially solubilized protein solution; and
a refolding agent containing, as the refolding agent, a low molecular weight monothiol or derivative thereof, these being selected from 2-mercaptoethanol, 3-mercaptopropionate, 2-mercaptoacetate, 2-mercaptoethylemine, cysteine, cysteamine and reduced glutathione, or mixtures thereof, said low molecular weight monothiol or derivative thereof being present in an amount effective to yield correctly disulphide bonded physiologically active protein; and
subjecting said at least partially solubilized protein solution to a refolding step by contacting said at least partially solubilized protein solution with said refolding agent so that said low molecular weight monothiol or derivative thereof is contacted with the solubilized protein in said at least partially solubilized protein solution in an amount effective to yield correctly disulphide bonded and physiologically active protein.

In a preferred form the method may include contacting the solubilised protein with the refolding agent in a molar ratio of refolding agent to protein of approximately 5 to 400 millimolar, preferably 5 to 200 millimolar, more preferably 45 to 65 millimolar at a protein concentration of approximately 0.05 to 10 mg/ml, more preferably 2.0 to 5.0 mg/ml. The refolding agent may be maintained in contact with the protein for an effective period during reformation of the disulphide bonds in the protein. The refolding agent may then be removed. However, it has surprisingly been found that such removal may not be necessiry.

It has surprisingly been found that treatment of the at least partially solubilised protein with a refolding agent, e.g. 2-mercaptoethanol, during refolding and disulphide bond formation, preferably in the molar ratio described above, significantly enhances the yield of correctly disulphide bonded (physiologically active) protein finally achieved. Improvements in yield of approximately 100% to 400% may be achievable utilising this technique.

Furthermore, it is surprisingly possible utilising the method of the present invention to optimally form correct protein disulphide bonds by air oxidation without the necessity of removal of the refolding agent.

Accordingly the method of the present invention may further include subjecting the solubilised protein to an oxidation step in the presence of the refolding agent to form correct protein disulphide bonds therein.

This is in contrast to the prior art which teaches the use of reducing agents such as 2-mercaptoethanol, Dithiothreitol (DDT) or Dithioerythritol (DTE) as agents for the breakage of protein disulphide bonds and/or their maintenance in a reduced (non-disulphide bonded) state; for example during or prior to SDS-gel electrophoresis. Where 2-mercaptoethanol has been used during the solubilisation or disulphide bonded recombinant proteins, the prior art teaches that substantially complete removal of 2-mercaptoethanol, for example via dialysis during protein refolding and reformation of protein disulphide bonds from the reduced state, is essential.

Preferably the oxidation step includes contacting the solubilised protein with an oxidation agent in the presence of a catalyst.

More preferably the oxidation agent is an ethanolamine-HCl mixture together with molecular oxygen and the catalyst is CuCl₂.

Reducing agents, such as Dithiothreitol (DTT) or Dithioerythritol (DTE) which are classed as "Dithiols", have not proved suitable as agents to enhance the yield of biologically active disulphide bonded protein during refolding.

It is clear from the results of the present invention that the controlled exposure of disulphide bond containing proteins to low molecular weight monothiols, including 2-mercaptoethanol, during refolding and concomitant disulphide bond formation, in fact provides an optimised in vitro environment that significantly lessens protein "aggregation" and polymerization to high molecular weight forms via otherwise undesirable inter-molecular disulphide bonding. This results in significantly increased yields of monomeric, correctly disulphide bonded (biologically active) protein.

Moreover, in contrast to the prior art which teaches that refolding of proteins, for example recombinant proteins at concentrations of less than or equal to 1 mg/ml and commonly 20 to 100 micrograms/ml is essential in order to maximise yields, we have found that when utilising the present invention we surprisingly obtain maximal yields of protein when refolding at protein concentrations of approximately 2 to 5 mg/ml or greater. This is of significant advantage over the prior art, particularly with regard to the operational ease and cost-effiency of producing kilogram quantities of, for example, recombinant proteins for use as veterinary therapeutics.

As stated above, low molecular weight monothiols such as cysteine, cysteamine and reduced glutathione; which are better known for their postulated roles as in vivo cellular reducing agents, have also been found to be suitable as agents to enhance correct disulphide bond formation in vitro. Such compounds are preferably used at concentrations in the range of from approximately 50 millimolar to 400 millimolar per 0.05 to 10 mg/ml of protein, and more preferably 100 to 200 millimolar as these tend to be less potent agents than the preferred monothiol mercaptans, such as 2-mercaptoethanol.

The source of protein in an at least partially solubilised form may be provided by the treatment of insoluble protein with a surfactant. Preferably the surfactant is a cationic surfactant such as described in Australian Patent 66874/86, the entire disclosure of which is incorporated herein by reference. The solubilised protein may accordingly be provided from a source of insoluble protein including bacterial inclusion bodies or any other protein aggregates.

The present invention is particularly applicable to biologically active proteins synthesised by micro-organisms and eukaryotic cell lines which have been modified by recombinant DNA technology. The protein aggregate may include an inclusion body isolated by disruption or lysis of a host cell which may have been transformed or transfected with a vector including a gene coding for the protein. However it is not restricted thereto. In addition the present invention is applicable to naturally occurring precipitated protein complexes.

Preferably the source of protein in a solubilised form is a source of protein aggregates solubilised with a cationic surfactant.

The protein aggregates which may be recovered according to the present invention may be selected from protein precipitates including inclusion bodies and cytoplasmic aggregates. The inclusion bodies may be selected from any suitable biologically active polypeptides and peptides including growth hormones including porcine, bovine and ovine growth hormones, interferons, immunogens and lymphokines.

For example, crude inclusion bodies including growth hormones in concentrations as low as approximately 10 to 12% may successfully be processed utilising the process of the present invention.

The refolding agents may be provided in any suitable form and in effective amounts. The low molecular weight monothiols may be present in amounts of from approximately 0.05% to 15% by weight based on the formula weight of the refolding agent. The refolding agent may be provided in a suitable aqueous solvent. A solution including a polar organic solvent may be used. Acetonitrile, or dimethyl sulphone may be used.

The refolding agent may further include an effective amount of a weak denaturing agent.

For example the weak denaturing agent may be selected from urea or derivatives thereof, including dimethylhydroxy urea, and dimethylsulphone and mixtures thereof. The weak denaturing agent may be present in an amount of from approximately 2.5 to 50% by weight, based on the formula weight of the refolding agent. The addition of a weak denaturant to the refolding agent further aids in increasing the yield of correctly disulphide bonded and therefore biologically active growth hormone.

In a preferred form where the refolding agent includes an effective amount of 2-mercaptoethanol and urea, the urea may be present at a concentration of approximately 0.5 to 5.0 molar, more preferably approximately 0.5 to 2 molar. Higher concentrations, for example in the range of approximately 4 to 5 molar are preferably used in refolding processes utilising dialysis, for example with water or an aqueous buffer solution.

In this form, the contacting step may continue for an extended period, for example for approximately 24 to 48 hours.

In a further preferred aspect of the present invention, the refolding agent may include a mixture of 2-mercaptoethanol and 2-hydroxyethyldisulphide as the low molecular weight monothiol. It will be understood that 2-hydroxyethyldisulphide is the oxidised form of 2-mercaptoethanol. Thus the use of 2-mercaptoethanol herein includes the use of a redox pair of 2-mercaptoethanol, that is, in equilibrium with 2-hydroxyethyldisulphide. The use of such a redox pair has been found to also increase the yield of physiologically active protein. The 2-mercaptoethanol and 2-hydroxyethyldisulphide may be provided in a molar ratio of 2-mercaptoethanol : 2-hydroxyethyldisulphide of approximately 0.5 : 1 to 5 : 1.

The refolding step may take any suitable form. The refolding step may include differential elution of the solubilised protein through a chromatographic column, dialysis, ultrafiltration, differential precipitation or ligand specific isolation. The chromatographic column may be a high performance liquid chromatography column (HPLC). Optionally a reversed phase HPLC column may be used. A column sold under the trade designation TSK-gel (LC) and available from Toyo Soda Manufacturing Co Ltd (Japan) or Ultrapore RPSC and available from Beckman Industries (California USA) have been found to be suitable. Other known forms of chromatography including chromatography of the molecular sieve type eg. gel filtration chromatography and ion exchange chromatography, hydrophobic interaction chromatography and ligand specific chromatography may be used.

The at least partially solubilised protein may be contacted with the refolding agent in any suitable manner during refolding. The protein and the refolding agent may be mixed directly. If desired the source of protein may be precipitated out of solution. The precipitate may then be mixed with the reducing or denaturing agent solution. However, it is not necessary to include a precipitation step. For example, the source of protein in a solubilised form may be diluted with or dialysed into a solution of the contacting agents.

The solubilised protein may during or prior to refolding be directly mixed with or dialysed against any appropriate aqueous buffer solution. A buffered saline solution ranging from approximately 0.1 to 1.5 molar may be used. The refolding agent may in whole or in part be added to the aqueous buffer solution. The aqueous buffer solution may include in addition to the low molecular weight monothiol, a weak denaturing agent selected from urea, dimethylsulphone and mixtures thereof, and an appropriate amount of 2-hydroxyethyldisulphide. In a preferred form the aqueous buffer solution may include urea in a concentration range of approximately 0.5 to 3 molar, preferably approximately 1 to 2 molar and 2-hydroxyethyldisulphide in a concentration range of approximately 5 millimolar to 500 millimolar.

Where a dialysis technique is used the solubilised protein may be dialysed during or prior to refolding against any suitable aqueous buffer solution including an effective amount of the refolding agent as described above. The protein is thereafter recovered in a biologically active disulphide bonded form by dialysis against an appropriate aqueous buffer solution, free of denaturing or reducing agents. Such a solution may be aerated or oxygenated to promote disulphide bond formation.

The source of surfactant solubilized protein may be utilised in a concentrated form. During solubilisation protein may be present in an amount of approximately 0.1 to 100 mg/ml, preferably approximately 20 to 30 mg/ml.

The method for the preparation of a protein in a physiologically active form may be conducted at any suitable temperature above the freezing point of the solution. Preferably a temperature in the range of approximately 4 to 37°C, more preferably 4 to 10°C may be used. At such relatively low temperatures an improved yield may be achieved.

Preferably, the pH of the solution is optimised to ensure solubility of the protein and stability of the solution. For example when the physiologically active protein is a growth hormone, the pH is maintained between approximately 8.5 and 11.0

Accordingly in a preferred aspect of the present invention there is provided a method for the preparation of a protein in a physiologically active form, which method comprises:
providing a source of protein in an at least partially solubilised form obtained by solubilising a water insoluble protein, obtained from inclusion bodies, with a cationic surfactant denaturing agent in the absence of additional denaturing agents to form at least partially solubilised protein solution, said protein being selected from the group of growth hormones, interferons, immunogenes and lymphokines, and a refolding agent containing, as the refolding agent, a low molecular weight monothiol or derivative thereof, these being selected from 2-mercaptoethanol, 3-mercaptopropionate, 2-mercaptoacetate, 2-mercaptoethylemine, cysteine, cysteamine and reduced glutathione, or mixtures thereof, said low molecular weight monothiol or derivative thereof being present in an amount effective to yield correctly disulphide bonded physiologically active protein;
exchanging the solvent of said at least partially solubilised protein solution with a second solvent selected from the group consisting of water and aqueous buffer solutions, optionally including a refolding agent containing, as the refolding agent, a low molecular weight monothiol or derivative thereof, these being selected from 2-mercaptoethanol, 3-mercaptopropionate, 2-mercaptoacetate, 2-mercaptoethylemine, cysteine, cysteamine and reduced glutathione or mixtures thereof; and
refolding said protein to its physiologically active form in said second solvent.

The method is particularly applicable to the production of disulphide bonded recombinant proteins in high yield.

It has been noted that in certain circumstances, the yield of correctly refolded proteins in biologically active form may further be enhanced by subjecting the protein to a further refolding or oxidation step. Accordingly in a preferred aspect the refolding step further includes subjecting the protein to a secondary refolding step in the presence of an oxidation agent and a catalyst such as the Cu²⁺ ion.

It has been found that although the presence of 2-mercaptoethanol or other monothiols increases the yield of currently refolded proteins, significant quantities (approximately 30% by HPLC) may, in certain embodiments, remain in a reduced (non-disulphide) bonded form after 24-48 hours of refolding.

The secondary refolding step may take any suitable form. The secondary refolding step may include a dialysis technique as described above. The dialysis may continue for a period of approximately 24 to 48 hours. The protein may be contacted with the oxidation agent in an aqueous buffer solution, such as a buffered saline solution, for example during dialysis.

The oxidation agent may be of any suitable type. An ethanolamine-HCl mixture using molecular oxygen with CuCl₂ has been found to be suitable.

The present invention will now be more fully described with reference to the accompanying examples. It should be understood, however, that the description following is illustrative only and should not be taken in any way as a restriction on the generality of the invention described above.

In the figures:

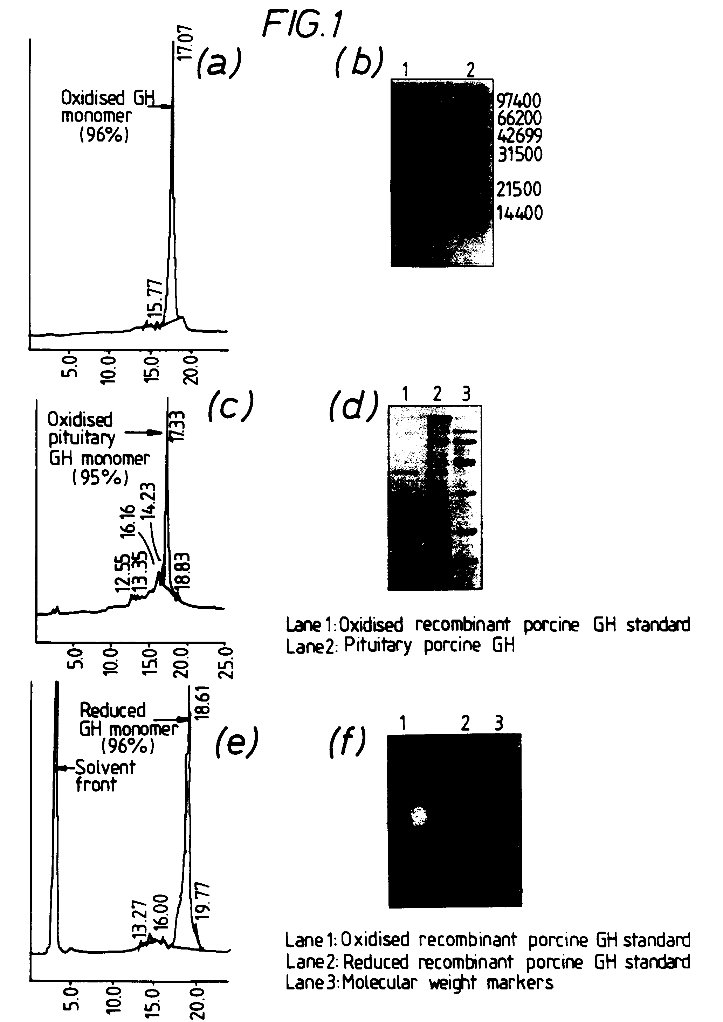

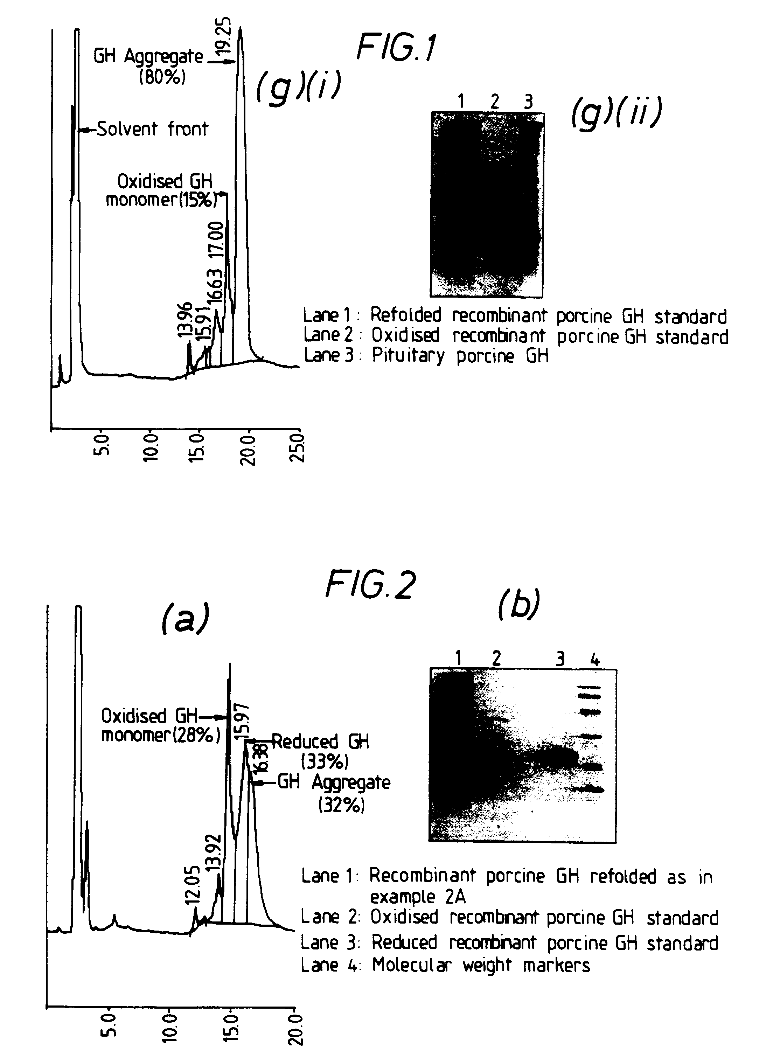

FIGURE 1 is a reverse-phase (RP) HPLC and SDS-PAGE analysis comparing various porcine growth hormone "standards" and the refolded recombinant growth hormone preparation as in Example 1.
- (a): RP-HPLC analysis of a "standard" preparation of refolded and fully biologically active (monomeric) recombinant porcine growth hormone purified by DE-52 ion exchange chromatography, and characterised by amino acid sequencing and a hyposectomised rat bioassay.
- (b): SDS-PAGE analysis of the above species of recombinant porcine growth hormone.
- (c): RP-HPLC analysis of purified porcine pituitary growth growth hormone prepared according to Methods in Enzymology, Vol. 37, p 360-380.
- (d): SDS-PAGE analysis of purified porcine pituitary growth hormone as above.
- (e): RP-HPLC analysis of reduced, that is non-disulphide bonded recombinant porcine growth hormone standard, prepared by treating the sample in (a) with 2% 2-mercaptoethanol for 1 hour in the presence of 3 M urea.
- (f): The same species as in (e) following SDS-PAGE analysis.
Note that the correctly disulphide bonded (monomeric) and the reduced (non-disulphide bonded) recombinant porcine growth hormones are clearly resolvable by their respective retention times on RP-HPLC (typically retention times differ by 1 minute) and by clear differences in mobility of SDS-PAGE.
- (g): A sample of recombinant porcine growth hormone solubilised and refolded as described in Example 1 and analysed by RP-HPLC (i) and SDS-PAGE (ii). The species corresponding to oxidised, monomeric recombinant growth hormone can be clearly identified as a peak eluting with the same retention time as the recombinant growth hormone and pituitary standards (see Figures 1(a) and 1(c)) and with the same mobility on SDS-PAGE (see Figures 1(b) and 1(d)). The proportion of the monomeric species as a percentage of the total peak area determined from the HPLC chromatogram is approximately 15%. The other major eluting species, with a peak retention time typically 1.5-2 minutes greater than that of the monomeric species corresponds to a polydisperse "aggregated", that is incorrectly disulphide bonded growth hormone fraction of varying molecular weight as evident from SDS-PAGE analysis (Part (ii)) of the refolded recombinant growth hormone preparation. The "aggregated" growth hormone fraction represents approximately 80% of the refolded species on RP-HPLC.

RP-HPLC was preformed using Cl alkyl-bonded silica columns (TSK-TMS 250; Toyo Soda Manufacturing Co., Tokyo, Japan; obtained through Pharmacia - LKB (Australia) Pty. Ltd.). Elution was preformed at a flow rate of 0.5 ml/min at room temperature with water/acetonitrile mixtures containing 0.1% (v/v) trifluoroacetic acid (TFA) as modifier. A stepwise linear gradient was constructed as follows: 100% buffer A (0.1% TFA in dH O) to 40% buffer B (100% acetonitrile, 0.1% TFA) in 10 minutes; 40% buffer B to 70% buffer B in 15 minutes; and to 100% buffer B in 5 minutes. The 100% acetonitrile, 0.1% TFA eluent was maintained for a further 0.5 minutes before re-equilibration, prior to the next injection. The composition of the solvent and flow rate may be varied slightly to achieve the desired resolution. All solution were degassed and filtered (0.4-5 m) and injection volumes of 20 ul were routinely used.

HPLC analysis was performed on a system comprising two Beckman Instruments 114M delivery system modules coupled to a 20 ul loop injector and a Beckman 421 controller. Detection was by U.V. at 280 nm fixed wavelength on a Beckman 165 Variable Wavelength detector.

SDS-PAGE analysis was preformed typically under non-reducing conditions using 15% polyacrylamide gels according to the discontinuous electrophoresis system of Laemmli; 1970 (Nature 227:p 680). Standard molecular weight markers are shown.

FIGURE 2 shows results of (a) RP-HPLC and (b) SDS-PAGE analysis (preformed as in Figure 1) of recombinant porcine growth hormone refolded in the presence of 55 millimolar 2-mercaptoethanol as described in Example 2(a).

The identity of oxidised (monomeric), reduced (non-disulphide bonded) and "aggregated" species of growth hormone was established as in Figure 1.

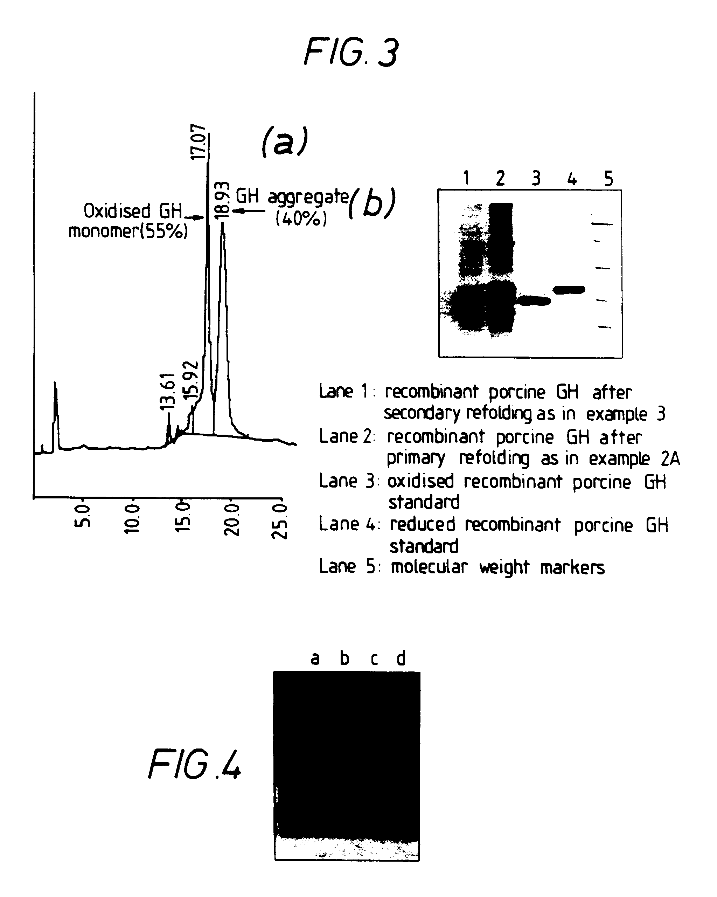

FIGURE 3 shows results of (a) RP-HPLC and (b) SDS-PAGE analysis (as in Figures 1 and 2) of recombinant growth hormone refolded as described in Example 3.

FIGURE 4 shows results of SDS-PAGE analysis of recombinant porcine growth hormone refolded as described in Example 5. Note the absence of major species of oxidised monomeric or reduced growth hormone as previously evident in Examples 1 to 4. In lane (a) 1 millimolar, (b) 5 millimolar, (c) 10 millimolar and lane (d) 20 millimolar DTT or DTE were used during refolding.

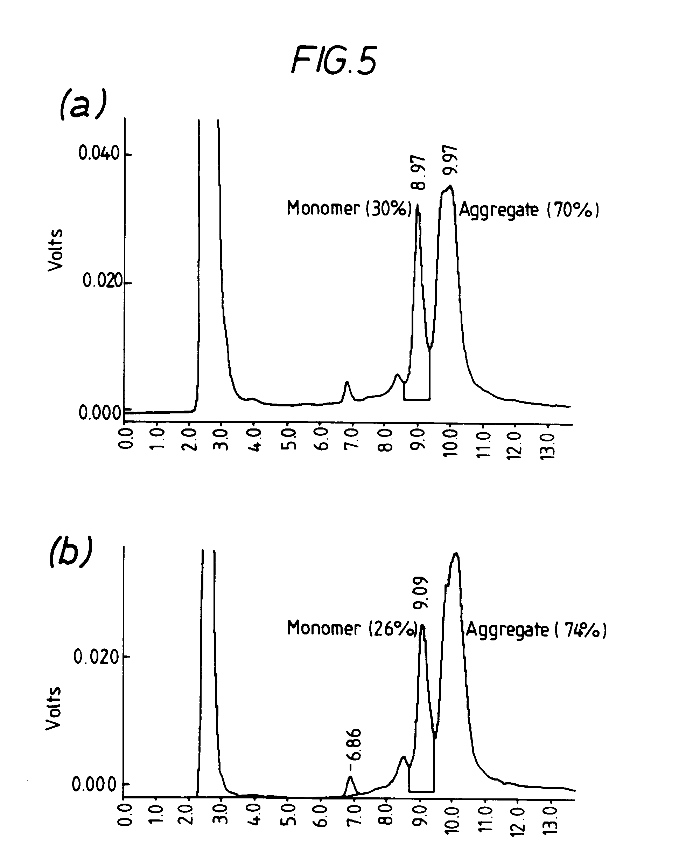

FIGURE 5 shows an inclusion body derived porcine growth hormone at a concentration of 1 mg/ml was allowed to refold in the presence of 55 mM 2-mercaptoethanol (A) or with the addition of 5 mM 2-hydroxyethyldisulfide (B). Refolding was monitored at 24 hours and 2 hours respectively by reverse phase high pressure liquid chromatography using a 280 nm absorbance detector.

With 2-hydroxyethyldisulfide in the refolding solution, 85% of the yield of native protein achievable by incubating in 2-mercaptoethanol alone, can be obtained in 2 hours.

### EXAMPLE 1 (Comparative)

### Solubilization and refolding of recombinant methionyl porcine growth hormone

Methionyl (1-190 amino acid) recombinant porcine growth hormone derived from plasmid pMG935 using the gene construct and prokaryotic expression system described in U.K. Patent Application No. 8701848, was produced in E.coli cells and solubilised from inclusion bodies using a cationic surfactant; essentially as described in U.S. Patent No. 4,797,474.

Inclusion bodies were isolated by cell disruption, harvested by differential centrifugation and washed with 0.1 M citric acid, 0.2 M disodium phosphate pH 4.0 (2 x) and distilled H₂O (2 x) prior to use. Inclusion bodies were used immediately or stored at pH 5 to 6.0 in a nitrogen purged atmosphere. Approximately 50 mg of inclusion bodies (117 mg/ml dry weight) were solubilised at 10 mg/ml protein in a solution of 0.1 M Tris-HCl pH 10.0, containing 2% mercaptoethanol (v/v) and 5% (w/v) cetyl trimethylammonium chloride for 1 hour at 55°C. The solubilised inclusion bodies were clarified by centrifugation (10,000 xg; 5 min.) and the supernatant fraction immediately mixed with 8 bed volumes of Dowex 50W x 4 (100 - 150 mesh) ion exchange resin (Dow Chemical Corporation, (U.S.A.)) equilibrated in 0.1 M Glycine-HCl and 5M urea, pH 10.0. (See Australian Patent Applications 11,412 and 15010). The solubilised, surfactant free, recombinant growth hormone at a protein concentration of approximately 1.5 mg/ml (based on dry weights of inclusion bodies) was subsequently dialysed against 20 millimolar ethanolamine-HCl pH 10.0 for 24-36 hours, in order to initiate refolding to obtain disulphide bonded (biologically active) growth hormone.

The refolded recombinant growth hormone preparation was subsequently analysed by reverse-phase HPLC in order to quantitate the proportion of correctly disulphide bonded (i.e., monomeric) and therefore biologically active species, as a percentage of the total monomer + "aggregated" forms; that is, the total growth hormone present after refolding.

The results presented in Figure 1(g) show that the above refolding procedure results in approximately 12 to 15% of the total growth hormone after refolding being present as the active monomeric species, while approximately 80% exists as a polydisperse "aggregate" species.

### EXAMPLE 2A

### Refolding of recombinant porcine growth hormone in the presence of a mercaptan; 2-mercaptoethanol

Solubilised and surfactant free, reduced, recombinant porcine growth hormone, obtained as in Example 1, at an approximate protein concentration of 1.5 mg/ml was exchanged using G-25 Sephadex gel filtration (PD-10 "desalting" columns obtained from Pharmacia-LKB (Australia) Pty. Ltd.) into a solution of 20mM ethanolamine-HCl pH 10.0 containing 2-mercaptoethanol at final concentrations of 5 millimolar, 45 millimolar, 75 millimolar and 100 millimolar. The resultant preparations were incubated for between 24 and 48 hours with shaking in an aerated environment at 4°C. Samples of refolded recombinant growth hormone were subsequently analysed by RP-HPLC and SDS-PAGE as described in Example 1.

The yield of monomeric, correctly disulphide bonded recombinant growth hormone as a percentage of the total area of peaks identified from the RP-HPLC chromatograms was respectively:
12% in the presence of 5 millimolar 2-mercaptoethanol;
25% in the presence of 45 millimolar 2-mercaptoethanol;
28% in the presence of 75 millimolar 2-mercaptoethanol; and
24% in the presence of 100 millimolar 2-mercaptoethanol.

The other major forms of recombinant growth hormone present after refolding in 45-65 millimolar 2-mercaptoethanol comprised a major reduced species and a lesser proportion of the "aggregated species. The presence of these forms of growth hormone was confirmed by SDS-PAGE.

Representative results comprising (a) the RP-HPLC chromatograms and (b) SDS-PAGE analysis of growth hormone refolded in the presence of the optimal 2-mercaptoethanol concentration (55 millimolar) are shown in Figure 2. Note the significant proportion of reduced (non-disulphide bonded) growth hormone present in comparison to the results obtained in Example 1 where the majority (80%) of the growth hormone existed as a polydisperse "aggregate" population.

### EXAMPLE 2B

### The effect of protein concentration and 2-mercaptoethanol on yield of monomeric growth hormone

Solubilised and surfactant free, reduced, recombinant porcine growth hormone, prepared as in Example 1, but at concentrations of approximately 1.5 mg/ml, 3.5 mg/ml and 7.5 mg/ml was prior to refolding exchanged by G-25 gel filtration (PD-10 columns; Pharmacia-LKB, Uppsalla, Sweden) into a solution of 20 millimolar ethanolamine HCl pH 10.0 containing 55 millimolar 2-mercaptoethanol. The resultant preparations were incubated for between 24 and 48 hours at 4°C with shaking in an aerated atmosphere. The yield of monomeric, correctly disulphide bonded, growth hormone was estimated as previously described by RP-HPLC. Yields of approximately 28%, 38% and 27% respectively for growth hormone refolded at 1.5 mg/ml, 3.5 mg/ml and 7.5 mg/ml were obtained. As previously found in Example 2A, the residual non-monomeric growth hormone existed mainly as a reduced (non-disulphide bonded species) and a lesser "aggregate" species.

It is clear from Examples 2A and 2B that both the concentration of protein and 2-mercaptoethanol are important in maximising the yield of correctly disulphide bonded growth hormone formed during refolding.

### EXAMPLE 3

### Secondary oxidation/refolding or porcine growth hormone

Examples 2A and 2B were used to demonstrate the effect of concentrations of protein and 2-mercaptoethanol on the yield of monomeric, disulphide bonded growth hormone, mainly at the expense of undesirable "aggregated" forms. However, the presence of residual reduced, that is non-disulphide bonded growth hormone (approximately 30% by RP-HPLC) after 24-48 hours of refolding in 55 millimolar 2-mercaptoethanol (whether at protein concentrations of 1.5, 3.5 or 7.5 mg/ml) suggested that an additional refolding step, that is, a two-stage procedure may further improve yield of growth hormone.

Porcine growth hormone from Example 2, refolded for 25 to 48 hours to obtain a yield of 38% of the monomeric species was exchanged via dialysis from buffer containing 2-mercaptoethanol into a solution of 20 millimolar ethanolamine-HCl containing 50 micromolar CuCl₂. The secondary refolding step was performed for betwen 24 to 36 hours and the dialysis solution aerated during this period. Yields of approximately 55% or growth hormone as the monomeric, correctly disulphide bonded form were obtained after secondary refolding as judged by RP-HPLC (Figure 3 (a)). The remaining growth hormone was present almost exclusively as the polydisperse "aggregate" form as judged by retention time on RP-HPLC and SDS-PAGE analysis (Figure 3(b)).

### EXAMPLE 4

### Refolding of porcine growth hormone in the presence of monothiols

Solubilised and surface free, reduced, porcine growth hormone prepared as in Example 1, at a protein concentration of approximately 3.5 mg/ml was prior to refolding exchanged by G-25 sephadex gel filtration (PD-10 columns; Pharmacia-LKB, Uppsalla, Sweden), into 20 mM ethanolamine-HCl pH 10.0, containing respectively 400, 200, 100 and 50 millimolar solutions of each of cysteamine or cysteine or reduced glutathione. The resultant preparations were incubated for between 24 to 36 hours with shaking at 4°C in an aerated atmosphere. Yields of monomeric oxidised GH were subsequently estimated by RP-HPLC. Optimal yields of approximately 30% monomeric growth hormone were found for cysteamine concentrations of 100 and 200 millimolar; cysteine concentrations of 50, 100 and 200 millimolar and reduced glutatione concentrations of 50 and 100 millimolar.

Secondary or two-stage refolding of the above growth hormone solutions as described in Example 3, resulted in an approximate yield of 40% of monomeric, correctly disulphide bonded, growth hormone.

### EXAMPLE 5 (Comparative)

### Refolding of porcine growth hormone in the presence of Dithiols

Solubilised and surfactant free, reduced, growth hormone prepared as in Example 1, at a protein concentration of 3.5 mg/ml or prepared by substituting 0.1 Molar DTT or DTE in place of the 2% 2-mercaptoethanol used during solubilisation (see Example 1) was, prior to refolding, exchanged using PD-10 columns (G-25 Sephadex Columns; Pharmacia-LKB, Uppsalla, Sweden) into a solution of 20 millimolar ethanolamine-HCl pH 10.0 containing DTT or DTE at concentrations of 0.5 millimolar, 5 millimolar, 10 millimolar and 20 millimolar respectively. The resultant preparations were incubated for between 24 to 36 hours under identical conditions to those described in Example 1, 2, 3, and 4 and yield of monomeric-oxidised growth hormone estimated as before by RP-HPLC. Based on the relative retention times of a purified recombinent porcine growth hormone standard (see Example 1) all the growth hormone present appeared as a broad eluting peak corresponding to the polydisperse aggregate form. The absence of significant amounts of either monomeric oxidised or reduced growth hormone as evident in Examples 1, 2, 3 and 4 was also confirmed by SDS-PAGE analysis, which showed a predominance of varying molecular weight (polydisperse) forms of porcine growth hormone (see Figure 4).

### EXAMPLE 6

### Refolding porcine growth hormone in the presence of a mercaptan and an appropriate concentration of a weak denaturant

Solubilised and surfactant free, reduced, growth hormone prepared as in Example 1, at concentrations of 1.5 or 3.4 or 7.5 mg/ml was, prior to refolding, exchanged via dialysis or gel filtration (PD-10); G-25 Sephadex COlumns; Pharmacia-LKB, Uppsalla, Sweden) into a solution of 20 millimolar ethanolamine-HCl pH 10.0, containing 55 millimolar 2-mercaptoethanol and urea; ranging from 0.5 molar to 5.0 molar. The resultant preparations were incubated for between 24 to 48 hours under conditions identical to those described in Example 1. Samples of each refolded growth hormone preparation were subsequently analysed by RP-HPLC and SDS-PAGE. Depending upon the concentration of urea used during refolding, two distinct effects were apparent:
- (i): In the presence of 55 millimolar 2-mercaptoethanol and for concentrations of urea between 0.5 to 2.0 molar, the yield of monomeric growth hormone as judged by RP-HPLC ranged from 30% to 40% (optimal of 40% at 1.0 M urea), with the residual growth hormone fraction comprising reduced (non-disulphide bonded) species and some "aggregate" forms as previously reported.
Note that at this stage, the yield of monomeric growth hormone may be further enhanced to approximately 50% by utilising the secondary oxidation procedure as described in Example 3. In the absence of Urea, the yield of monomeric growth hormone was approximately 28% as in Example 2A. Further, the yield of monomeric growth hormone in the presence of 0.5 to 2.0 M Urea was found to be independent of the concentration of protein used during refolding (i.e. 1.5 mg/ml or 3.5 mg/ml or 7.5 mg/ml) in contrast to the results described in Example 2B.
- (ii): At concentrations of urea between 2.0 and 5.0 molar, the results of both RP-HPLC and SDS-PAGE surprisingly showed that the majority of the growth hormone had failed to undergo oxidation, existing consequently in a reduced (non-disulphide bonded) state. For example at urea concentrations of between 2 and 3 molar, approximately 75% to 80% and, at urea concentrations of between 4 and 5 molar, approximately 95% of the growth hormone was found to exist as a single, fully reduced species.
This can be thought of as a "poised" state.
In order to oxidise this species, the secondary or two stage oxidation procedure utilising 20 millimolar ethanolamine-HCl pH 10.0 and CuCl₂ as described in Example 3, was used. After this procedure, approximately 50 to 55% of the growth hormone was present in a monomeric, oxidised form and approximately 40 to 50% as the "aggregated" species. The above effect was as in part (i) independent of the concentration of growth hormone used during refolding.
The results in this example show that appropriate concentrations of urea, optimally either 0.5 to 2.0 molar or 4 to 5 molar, may be used to augment the yield enhancing effects of 2-mercaptoethanol on monmeric growth hormone; particularly under circumstances where only low concentrations (1.5 to 3.5 mg/ml or less) of recombinant protein are available for refolding. (see Example 2B which illustrates the effect of protein concentration on the yield of monomeric growth hormone.).

### EXAMPLE 7

### Refolding porcine growth hormone in the presence of 2-mercaptoethanol and 2-hydroxyethyldisulphide

Solubilised and surfactant free, reduced, growth hormone prepared as in Example 1, at concentrations of approximately 3.5 mg/ml was, prior to refolding, exchanged via gel filtration (PD-10); G-25 Sephadex Columns; Pharmacia-LKB, Uppsalla, Sweden) into a solution of 20 millimolar ethanolamine-HCl pH 10.0, containing 55 millimolar 2-mercaptoethanol and 2-hydroxyethyl disulphide ranging in concentration from 5 millimolar to 100 millimolar. The resultant preparations were incubated for between 24 and 48 hours as in Example 1, and samples of each refolded growth hormone preparation subsequently analysed by RP-HPLC and SDS-PAGE.

Yield of monomeric growth hormone of approximately 25 to 40% were found depending upon the concentration of 2-hydroxyethyl disulphide used. The optimal concentration range for 2-hydroxyethyl disulphide was from about 5 millimolar to 100 millimolar with 5 to 50 mM being preferred. Residual amounts of reduced growth hormone could, as previously described in Example 3, be subjected to secondary oxidation to bring yields of monomeric growth hormone to approximately 45-50% of the solubilised growth hormone present on RP-HPLC.

### EXAMPLE 8

### Accelerated refolding of porcine growth hormone in the presence of 2-mercaptoethanol and 2-hydroxyethyldisulfide

Solubilised and surfactant free, reduced, porcine growth hormone prepared as in Example 1, was prior to refolding exchanged by G-25 sephadex gel filtration (PD-10 columns; Pharmacia-LKB, Uppsalla, Sweden), into 20 mM ethanolamine-HCL pH 10.0, containing 55 millimolar 2-mercaptoethanol with or without 5 - 40 millimolar 2-hydroxyethyldisulfide. The resultant preparations had a protein concentration of 1 mg/ml and were incubated for 24 hours and 2 hours respectively with shaking at 4°C in an aerated atmosphere. Yields of monomeric oxidised GH were estimated by RP-HPLC. Approximately 85% of the nature protein which was recovered in 24 hours by incubation in 2-mercaptoethanol alone, was recovered in 2 hours by using 2-hydroxyethyldusulfide (see Figure 5).

Finally, it is to be understood that various other modifications and/or alterations may be made without departing from the spirit of the present invention as outlined herein.

## Claims

1. A method of preparing a protein in a physiologically active form, which method includes
providing
a source of protein in an at least partially solubilized form obtained by solubilizing an insoluble protein with a cationic surfactant denaturing agent in the absence of additional denaturing agents to form an at least partially solubilized protein solution; and
a refolding agent containing, as the refolding agent, a low molecular weight monothiol or derivative thereof, these being selected from 2-mercaptoethanol, 3-mercaptopropionate, 2-mercaptoacetate, 2-mercaptoethylemine, cysteine, cysteamine and reduced glutathione, or mixtures thereof, said low molecular weight monothiol or derivative thereof being present in an amount effective to yield correctly disulphide bonded physiologically active protein; and
subjecting said at least partially solubilized protein solution to a refolding step by contacting said at least partially solubilized protein solution with said refolding agent so that said low molecular weight monothiol or derivative thereof is contacted with the solubilized protein in said at least partially solubilized protein solution in an amount effective to yield correctly disulphide bonded and physiologically active protein.

2. A method according to claim 1, wherein said low molecular weight monothiol is 2-mercaptoethanol.

3. A method according to claim 2, wherein the refolding step includes contacting said at least partially solubilized protein solution at a concentration of approximately 0.5 to 10 mg solubilized protein/ml with said refolding agent to form refolded proteins, said refolding agent including said low molecular weight monothiol at a concentration of approximately 5 to 400mM.

4. A method according to claim 3, further including subjecting the refolded proteins to a secondary refolding step.

5. A method according to claim 4, wherein the secondary refolding step includes further subjecting the refolded proteins to an oxidation step.

6. A method according to claim 5, wherein the oxidation agent is molecular oxygen and CuCl₂ is used as a catalyst.

7. A method according to any preceding claim, wherein the source of protein includes protein aggregates including inclusion bodies isolated from a host cell which has been transformed or transfected with a vector including a gene coding for the protein.

8. A method according to claim 7, wherein said refolding agent includes:
approximately 0.05 to 15% by weight based on the total weight of the refolding agent, of said low molecular weight monothiol or derivative thereof; and
approximately 2.5 to 50% by weight based on the total weight of the refolding agent, of a weak denaturing agent selected from urea, dimethylsulphone, and mixtures thereof.

9. A method for the preparation of a protein in a physiologically active form, which method comprises:
providing a source of protein in an at least partially solubilised form obtained by solubilising a water insoluble protein, obtained from inclusion bodies, with a cationic surfactant denaturing agent in the absence of additional denaturing agents to form at least partially solubilised protein solution, said protein being selected from the group of growth hormones, interferons, immunogenes and lymphokines, and a refolding agent containing, as the refolding agent, a low molecular weight monothiol or derivative thereof, these being selected from 2-mercaptoethanol, 3-mercaptopropionate, 2-mercaptoacetate, 2-mercaptoethylemine, cysteine, cysteamine and reduced glutathione, or mixtures thereof, said low molecular weight monothiol or derivative thereof being present in an amount effective to yield correctly disulphide bonded physiologically active protein;
exchanging the solvent of said at least partially solubilised protein solution with a second solvent selected from the group consisting of water and aqueous buffer solutions, optionally including a refolding agent containing, as the refolding agent, a low molecular weight monothiol or derivative thereof, these being selected from 2-mercaptoethanol, 3-mercaptopropionate, 2-mercaptoacetate, 2-mercaptoethylemine, cysteine, cysteamine and reduced glutathione or mixtures thereof; and
refolding said protein to its physiologically active form in said second solvent.

## Patentansprüche

1. Verfahren zur Herstellung eines Proteins in einer physiologisch aktiven Form, welches [folgende Stufen] umfaßt:
Bereitstellung
einer Proteinquelle in einer mindestens teilweise solubilisierten Form, erhalten durch Solubilisierung eines unlöslichen Proteins mit einem kationischen Tensid-Denaturierungsmittel in Abwesenheit von zusätzlichen Denaturierungsmitteln, um eine Lösung eines mindestens teilweise solubilisierten Proteins zu erhalten; und
eines Rückfaltemittels, das als Rückfaltemittel einen niedrigmolekularen Monothiol oder ein Derivat hiervon enthält, welche ausgewählt sind aus 2-Mercaptoethanol, 3-Mercaptopropionat, 2-Mercaptoacetat, 2-Mercaptoethylemin, Cystein, Cysteamin und reduziertem Glutathion oder deren Gemischen, wobei der niedrigmolekulare Monothiol oder sein Derivat in einer Menge vorhanden sind, die wirksam ist, um korrekt disulfidverknüpftes, physiologisch aktives Protein zu ergeben; und
Behandlung der Lösung des mindestens teilweise solubilisierten Proteins in einer Rückfaltestufe durch Kontaktieren der Lösung des mindestens teilweise solubilisierten Proteins mit dem Rückfaltemittel, so daß der niedrigmolekulare Monothiol oder sein Derivat mit dem solubilisierten Protein in der Lösung des mindestens teilweise solubilisierten Proteins kontaktiert wird in einer Menge, die wirksam ist, um korrekt disulfidverknüpftes und physiologisch aktives Protein zu ergeben.

2. Verfahren nach Anspruch 1, worin der niedrigmolekulare Monothiol 2-Mercaptoethanol darstellt.

3. Verfahren nach Anspruch 2, worin die Rückfaltestufe die Kontaktierung der Lösung des mindestens teilweise solubilisierten Proteins in einer Konzentration von etwa 0,5 bis 10 mg solubilisiertem Protein/ml mit dem Rückfaltemittel umfaßt, um rückgefaltete Proteine zu erzeugen, wobei das Rückfaltemittel den niedrigmolekularen Monothiol in einer Konzentration von etwa 5 bis 400 mM enthält.

4. Verfahren nach Anspruch 3, worin die rückgefalteten Proteine einem zweiten Rückfalteschritt unterzogen werden.

5. Verfahren nach Anspruch 4, worin der zweite Rückfalteschritt die Behandlung der rückgefalteten Proteine in einer weiteren Oxidationsstufe umfaßt.

6. Verfahren nach Anspruch 5, worin das Oxidationsmittel molekularen Sauerstoff darstellt und CuCl₂ als Katalysator verwendet wird.

7. Verfahren nach einem der vorhergehenden Ansprüche, worin die Proteinquelle Proteinaggregate enthält, die Einschlußkörper enthalten, welche aus einer Wirtszelle isoliert wurden, welche mit einem Vektor, einschließlich eines Gens, das für das Protein codiert, transformiert oder transfiziert worden ist.

8. Verfahren nach Anspruch 7, worin das Rückfaltemittel enthält:
etwa 0,05 bis 15 Gew.-%, bezogen auf das Gesamtgewicht des Rückfaltemittels, des niedrigmolekularen Monothiols oder seines Derivats; und
etwa 2,5 bis 50 Gew.-%, bezogen auf das Gesamtgewicht des Rückfaltemittels, eines schwachen Denaturierungsmittels, ausgewählt aus Harnstoff, Dimethylsulfon und deren Gemischen.

9. Verfahren zur Herstellung eines Proteins in einer physiologisch aktiven Form, welches [folgende Stufen] umfaßt:
Bereitstellung einer Proteinquelle in einer mindestens teilweise solubilisierten Form, erhalten durch Solubilisierung eines wasserunlöslicnen Proteins, erhalten aus Einschlaßkörpern, mit einem kationischen Tensid-Denaturierungsmittel in Abwesenheit von zusätzlichen Denaturierungsmitteln, um eine Lösung eines mindestens teilweise solubilisierten Proteins zu erhalten, wobei das Protein ausgewählt ist aus der Gruppe der Wachstumshormone, Interferone, Immunogene und Lymphokine, und eines Rückfaltemittels, das als Rückfaltemittel einen niedrigmolekularen Monothiol oder ein Derivat hiervon enthält, welche ausgewählt sind aus 2-Mercaptoethanol, 3-Mercaptopropionat, 2-Mercaptoacetat, 2-Mercaptoethylemin, Cystein, Cysteamin und reduziertem Glutathion oder deren Gemischen, wobei der niedrigmolekulare Monothiol oder sein Derivat in einer Menge vorhanden sind, die wirksam ist, um korrekt disulfidverknüpftes, physiologisch aktives Protein zu ergeben;
Austauschen des Lösungsmittels der Lösung des mindestens teilweise solubilisierten Proteins gegen ein zweites Lösungsmittel, ausgewählt aus der Gruppe, bestehend aus Wasser und wäßrigen Pufferlösungen, welche gegebenenfalls ein Rückfaltemittel enthalten, das als Rückfaltemittel einen niedrigmolekularen Monothiol oder ein Derivat hiervon enthält, welche aus 2-Mercaptoethanol, 3-Mercaptopropionat, 2-Mercaptoacetat, 2-Mercaptoethylemin, Cystein, Cysteamin und reduziertem Glutathion oder deren Gemischen ausgewählt sind; und
Rückfaltung des Proteins in seine physiologisch aktive Form in dem zweiten Lösungsmittel.

## Revendications

1. Procédé de préparation d'une protéine sous une forme physiologiquement active, ledit procédé comprenant les étapes consistant à :
fournir une source de protéine sous une forme au moins partiellement solubilisée obtenue par solubilisation d'une protéine insoluble avec un agent dénaturant du type agent tensio-actif cationique en l'absence d'agents dénaturants supplémentaires ; et
un agent de repliement contenant, comme agent de repliement, un monothiol de faible poids moléculaire ou un dérivé de celui-ci, ceux-ci étant choisis parmi le 2-mercaptoéthanol, le 3-mercaptopropionate, le 2-mercaptoacétate, la 2-mercaptoéthylamine, la cystéine, la cystéamine et un glutathion réduit, ou des mélanges de ceux-ci, ledit monothiol de faible poids moléculaire ou le dérivé de celui-ci étant présent en une quantité efficace pour donner une protéine physiologiquement active correctement liée à un disulfure ; et
soumettre ladite solution de protéine au moins partiellement solubilisée à une étape de repliement en amenant ladite solution de protéine au moins partiellement solubilisée au contact dudit agent de repliement de telle façon que ledit monothiol de faible poids moléculaire ou le dérivé de celui-ci soit au contact de la protéine solubilisée dans ladite solution de protéine au moins partiellement solubilisée, en une quantité efficace pour donner une protéine correctement liée à un disulfure et physiologiquement active.

2. Procédé selon la revendication 1, dans lequel ledit monothiol de faible poids moléculaire est le 2-mercaptoéthanol.

3. Procédé selon la revendication 2, dans lequel l'étape de repliement comprend la mise en contact de ladite solution de protéine au moins partiellement solubilisée, à une concentration d'environ 0,5 à 10 mg de protéine solubilisée/ml, avec ledit agent de repliement pour former des protéines repliées, ledit agent de repliement comprenant ledit monothiol de faible poids moléculaire à une concentration d'environ 5 à 400 mM.

4. Procédé selon la revendication 3, comprenant, en outre, la soumission des protéines repliées à une étape de repliement secondaire.

5. Procédé selon la revendication 4, dans lequel l'étape de repliement secondaire comprend, en outre, la soumission des protéines repliées à une étape d'oxydation.

6. Procédé selon la revendication 5, dans lequel l'agent d'oxydation est de l'oxygène moléculaire, et on utilise du CuCl₂ comme catalyseur.

7. Procédé selon l'une quelconque des revendications précédentes, dans lequel la source de protéine comprend des agrégats de protéines comprenant des corps d'inclusion isolés d'une cellule hôte qui a été transformée ou transfectée avec un vecteur comprenant un gène codant pour la protéine.

8. Procédé selon la revendication 7, dans lequel ledit agent de repliement comprend :
environ 0,05 à 15 % en poids dudit monothiol de faible poids moléculaire ou du dérivé de celui-ci, par rapport au poids total d'agent de repliement, et
environ 2,5 à 50 % en poids d'un agent faiblement dénaturant choisi parmi l'urée, la diméthylsulfone et des mélanges de celles-ci, par rapport au poids total d'agent de repliement.

9. Procédé de préparation d'une protéine sous une forme physiologiquement active, ledit procédé comprenant les étapes consistant à :
fournir une source de protéine sous une forme au moins partiellement solubilisée, obtenue par solubilisation d'une protéine insoluble dans l'eau, obtenue à partir de corps d'inclusion, avec un agent dénaturant du type agent tensio-actif cationique, en l'absence d'agents dénaturants supplémentaires, pour former une solution de protéine au moins partiellement solubilisée, ladite protéine étant choisie dans l'ensemble constitué par les hormones de croissance, les interférons, les immunogènes et les lymphokines, et un agent de repliement contenant, comme agent de repliement, un monothiol de faible poids moléculaire ou un dérivé de celui-ci, ceux-ci étant choisis parmi le 2-mercaptoéthanol, le 3-mercaptopropionate, le 2-mercaptoacétate, la 2-mercaptoéthylamine, la cystéine, la cystéamine et un glutathion réduit, ou des mélanges de ceux-ci, ledit monothiol de faible poids moléculaire ou le dérivé de celui-ci étant présent en une quantité efficace pour donner une protéine active correctement liée à un disulfure et physiologiquement active ;
le remplacement du solvant de ladite solution de protéine au moins partiellement solubilisée par un second solvant choisi dans l'ensemble constitué par l'eau et des solutions aqueuses tamponnées, comprenant éventuellement un agent de repliement contenant, comme agent de repliement, un monothiol de faible poids moléculaire ou un dérivé de celui-ci, ceux-ci étant choisis parmi le 2-mercaptoéthanol, le 3-mercaptopropionate, le 2-mercaptoacétate, la 2-mercaptoéthylamine, la cystéine, la cystéamine et un glutathion réduit ou des mélanges de ceux-ci ; et
le repliement de ladite protéine en sa forme physiologiquement active dans ledit second solvant.
